(19) Europäisches Patentamt / European Patent Office / Office européen des brevets

(11) EP 4 751 719 A1

(12) **EUROPEAN PATENT APPLICATION**

(43) Date of publication:
03.06.2026  Bulletin 2026/23

(21) Application number: 24216569.4

(22) Date of filing: **29.11.2024**

(51) International Patent Classification (IPC):
*A61K 9/72* (2006.01)     *A61K 47/10* (2017.01)
*A61K 47/26* (2006.01)    *A61K 47/32* (2006.01)
*A61K 47/42* (2017.01)    *A61K 31/137* (2006.01)
*A61K 31/167* (2006.01)   *A61K 31/439* (2006.01)
*A61K 31/46* (2006.01)    *A61K 31/5377* (2006.01)
*A61K 9/16* (2006.01)

(52) Cooperative Patent Classification (CPC):
A61K 9/0075; A61K 31/137; A61K 31/167;
A61K 31/439; A61K 31/46; A61K 31/5377;
A61K 47/10; A61K 47/26; A61K 47/32; A61K 47/42

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Steinbeis Transfer GmbH**
**70599 Stuttgart (DE)**

(72) Inventors:
• **LAMPRECHT, Alf**
**53121 Bonn (DE)**
• **GROSS, Roman**
**53121 Bonn (DE)**
• **RAUTENBERG, Annika**
**53121 Bonn (DE)**
• **VAZQUEZ-OLVERA, Jose Ignacio**
**53121 Bonn (DE)**

(74) Representative: **Maiwald GmbH**
**Elisenhof**
**Elisenstraße 3**
**80335 München (DE)**

(54) **SPRAY-FREEZE-DRIED PHARMACEUTICAL PULMONARY POWDER CAPABLE OF FRAGMENTATION UPON INSPIRATION**

(57)    The present application relates to spray-freeze-dried pulmonary powders with good handling properties due to greater initial particles sizes that fragment on their way into the lungs. In particular, the present disclosure relates to a spray-freeze-dried pharmaceutical powder for inhalation comprising solid particles of at least one excipient and at least one active agent, wherein the maximum of the particle size distribution of the particles is higher than 150 $\mu$m, wherein the particles are fragmented to pulmonary particles during inhalation with a median aerodynamic diameter of below 10 $\mu$m.

Figure 3

**Description**

BACKGROUND OF THE INVENTION

Field of the invention

The present invention is in the field of pulmonary drug delivery. Doses of pulmonary drugs may be delivered by dry powder inhalers. The present application relates to a spray-freeze-dried pulmonary powder with good handling properties and the use of said powder in the treatment or the prevention of a pulmonary disease. The invention further relates to capsules commonly used in dry powder inhalers that are filled with the powder of present invention.

Background

Pulmonary drug delivery has emerged as a critical approach for administering therapeutic agents directly to the lungs, offering advantages in treating pulmonary diseases such as asthma, chronic obstructive pulmonary disease (COPD), cystic fibrosis, and pulmonary infections. This route allows rapid drug action and targeted delivery, often with reduced systemic side effects compared to oral or injectable routes. However, developing powder particles for effective pulmonary drug delivery remains technically challenging, especially given the requirements for particle size and aerodynamic properties necessary for efficient lung deposition. Specifically, the handling and flowability of very small particles for pulmonary application presents significant challenges in both manufacturing and delivery.

One of the main difficulties with small particle formulations is achieving consistent flowability. Fine particles tend to exhibit poor flow characteristics due to their high surface area-to-volume ratio, which increases the likelihood of particle-particle interactions, including adhesion, cohesion, and aggregation. These interactions can lead to clumping and inconsistent dosing, as particles may stick together rather than flow freely. This poor flowability complicates the manufacturing process, making it difficult to ensure uniform mixing, accurate dosing, and efficient filling into inhaler devices or capsules. As a result, achieving reproducible doses becomes a challenge, which may impact the efficacy and safety of the treatment for patients.

Furthermore, the electrostatic properties of very small particles exacerbate handling issues. Fine particles are prone to developing static charges, especially during processes involving mechanical agitation or pneumatic transfer, such as during the filling of inhalation devices. Electrostatic charges cause particles to adhere to container walls or device components, creating variations in the dose delivered. In some cases, these charges can lead to substantial loss of the drug within the delivery device, reducing the effectiveness of the therapy and leading to inconsistent patient experiences.

In addition to handling challenges, poor flowability can negatively impact the aerosolization performance of pulmonary drug delivery systems. Efficient aerosolization requires that particles are easily dispersible within an air stream for effective inhalation. However, highly cohesive particles tend to resist dispersion, leading to lower respirable fractions and inefficient delivery to the lower respiratory tract. As a result, a significant portion of the drug may deposit in the oropharyngeal region rather than reaching the target sites in the lungs, limiting therapeutic effectiveness and increasing the likelihood of side effects.

Current approaches to improve flowability and handling, such as using excipients to modify surface properties, coating particles, or agglomerating particles into larger, more manageable forms had limited success. These methods often involve additional processing steps, which can be costly, time-consuming, and may even compromise drug stability or bioavailability. Additionally, some of these techniques introduce excipients or carriers that may not be biocompatible, especially for sensitive lung tissues, potentially causing irritation or adverse reactions in patients.

Given these limitations, there is a clear need for innovative particle formulations and processing methods that enhance the flowability and handling of very small particles for pulmonary application, while maintaining their stability, dispersibility, and safety for inhalation use. Improvements in these areas would advance the field of pulmonary drug delivery by ensuring consistent dosing, improving lung deposition efficiency, and reducing variability in patient outcomes.

Powder drugs delivered through inhalers are carefully engineered to target specific regions within the pulmonary system, including the nasal passages, throat, and various segments of the lungs such as the bronchi, bronchioles, and alveolar regions. The effectiveness of this targeting hinges on the aerodynamic properties of the particles.

SUMMARY

The present application relates to spray-freeze-dried pulmonary powders with good handling properties due to greater initial particles sizes, which particles fragment on their way into the lungs. The present disclosure relates in particular to an improved powder suitable for pulmonary drug delivery.

In a first aspect the disclosure relates to a spray-freeze-dried pharmaceutical powder for inhalation, the powder comprising solid particles of at least one excipient and at least one active agent; wherein the maximum of the particle size distribution is higher than 150 μm; and wherein the particles are fragmented during inhalation to particles with a median aerodynamic diameter of below 10 μm.

In a second aspect the disclosure relates to the powder according to present disclosure for use in the treatment or the prevention of a pulmonary disease, preferably wherein the pulmonary disease is selected from one or more elements of the group consisting of Asthma, Chronic Obstructive Pulmonary Disease (COPD), Pulmonary Fibrosis, Cystic Fibrosis, Pulmonary Hypertension, Bronchiectasis, Pneumonia, Tuberculosis (TB), Acute Respiratory Distress Syndrome (ARDS), Lung Cancer, Pulmonary Embolism (PE), Sarcoidosis, Interstitial Lung Disease (ILD), Alpha-1 Antitrypsin Deficiency, Bronchiolitis Obliterans (Popcorn Lung), Hypersensitivity Pneumonitis, Pleural Effusion, Asbestosis, Respiratory Syncytial Virus (RSV) Infection.

In a third aspect the disclosure relates to a capsule suitable for provision in an inhalation system comprising the powder according to present invention.

The inventors of present disclosure surprisingly and unexpectedly found spray-freeze-dried particles according to present disclosure to be suitable for pulmonary administration with an initial particles size distribution above 150 μm that is usually unsuitable to reach the deeper lungs as required for pulmonary administration.

BRIEF DESCRIPTION OF THE DRAWINGS

Fig. 1: SEM images of various excipients at a solid content of 5 %; upper scale bar represents 200 μm, bottom scale bar 1000 μm.

Fig. 2: Analysis of lung deposition in NGI experiments for various excipients in 5 % solid content.

Fig. 3: SEM image of mannitol in various solid contents; scale bar top row: 200 μm, bottom row: 1000 μm.

Fig. 4: Analysis of lung deposition in NGI experiments of mannitol at different excipient concentrations; FPF values for solid content 5-20 %.

Fig. 5: Analysis of lung deposition in NGI experiments of mannitol in combination with salbutamol (0.6% in the spraying solution) at different excipient concentrations.

Fig. 6: SEM images of spray-freeze-dried powders at 3-5 % solid content, with salbutamol (0.6 % in the spraying solution) and mannitol or pure salbutamol.

Fig. 7: Overview of FPFs for various solid contents and excipients, as well as active agents.

Fig. 8: 3D printed inlays: 8A Inlay 2, 8B Inlay 3.

Fig. 9: Analysis of lung deposition in NGI experiments with 5 % solid content mannitol and placebo fluorescein without and with 3D printed inlays 1 and 2.

Fig. 10: Particle density (A, x-axis cut off at 100 μm for better differentiation) and cumulative particle size distribution (B, full range) of mannitol 5 % in free fall (grey) and various dispersion pressures (logarithmic colour scale) for the determination of suitable analysis parameters for breakability measurements.

Fig. 11: SEM images of 5 % solid content mannitol with fluorescein on different stages of an NGI experiment.

Fig. 12: Aerodynamic particle size distributions in NGI experiments of mannitol-salbutamol particles with a total solid content of 5 % with varying proportions of mannitol to salbutamol; particles with 5 % solid content of salbutamol concentrations of 10 %, 25 % and 50 %.

Fig. 13: SEM images of mannitol-salbutamol particles tested in NGI experiments displayed in Fig. 12, Example 6

Fig. 14: Particle size distribution in NGI experiment of 5 % insoluble aprepitant nanocrystals and 5 % mannitol; total solid content of 10 %.

Fig. 15: Overview of analysis of lung deposition in NGI experiments of mannitol at different excipient concentrations; FPF values for solid content 5-20 %.

DETAILED DESCRIPTION

In a first aspect, the present disclosure relates to a spray-freeze-dried pharmaceutical powder for inhalation comprising solid particles comprising at least one excipient and at least one active agent, wherein the maximum of the particle size distribution is higher than 150 $\mu$m, wherein the particles are fragmented to pulmonary particles during inhalation with a median aerodynamic diameter of below 10 $\mu$m.

The particles size of the solid particles of the powder refers to the particles before fragmentation to pulmonary particles during inhalation.

In pulmonary drug delivery, the particle size of a particle loaded with an active agent plays a critical role in determining the efficiency of drug deposition in the lungs and the ease of handling during manufacturing.

The powder comprising solid particles according to the present disclosure enhances the manufacturability and consistency by reducing cohesion and electrostatic issues due to an increased particle size, while still allowing effective pulmonary deposition by fragmentation to a smaller particle size that is therefor suitable. The powder shows an improvement in balancing ease of handling due to an increased flowability (<45 °, measured via angle of repose) and therapeutic efficacy.

The aerodynamic diameter of inhaled particles plays a critical role in determining their deposition within the respiratory tract. The aerodynamic diameter is defined as the diameter of a spherical reference particle with a density of 1 g/cm$^3$ that settles in still air at the same velocity as the actual particle (Equation 3). This measure is significant because it accounts for both the physical size and the density of particles, which together influence how particles behave aerodynamically in the lungs.

A small aerodynamic diameter is advantageous over merely small particle size due to its enhanced potential for deep lung penetration and efficient deposition. Particles with an aerodynamic diameter of below 10 $\mu$m are generally optimal for reaching the deeper lungs and alveolar regions, where they can be absorbed into the bloodstream.

In a second aspect, the present disclosure relates to a pharmaceutical powder for use in the treatment of a pulmonary disease, preferably selected from the group of Asthma, Chronic Obstructive Pulmonary Disease (COPD), Pulmonary Fibrosis, Cystic Fibrosis, Pulmonary Hypertension, Bronchiectasis, Pneumonia, Tuberculosis (TB), Acute Respiratory Distress Syndrome (ARDS), Lung Cancer, Pulmonary Embolism (PE), Sarcoidosis, Interstitial Lung Disease (ILD), Alpha-1 Antitrypsin Deficiency, Bronchiolitis Obliterans (Popcorn Lung), Hypersensitivity Pneumonitis, Pleural Effusion, Asbestosis, Respiratory Syncytial Virus (RSV) Infection.

In treating pulmonary diseases, localized pulmonary drug delivery allows for precise targeting, enabling faster therapeutic action with lower doses, and significantly reduces systemic side effects. This approach minimizes drug interactions and lowers the risk of adverse effects on non-target organs, enhancing patient safety. Additionally, inhalation therapies are often non-invasive, improving patient comfort and compliance.

The present disclosure relates to a locally pulmonary-effective pharmaceutical powder that offers an efficient, patient-friendly, and safer alternative for treating pulmonary diseases. The powder according to present disclosure allows for an improved dosing accuracy and consistency, as well as a uniform distribution of the active agent due to the increased particle size that reduces cohesion and electrostatic forces.

In a third aspect, the present disclosure relates to capsule suitable for provision in an inhalation system comprising the powder according to the present invention.

Encapsulating pulmonary powders for inhalation systems provides distinct advantages that enhance the effectiveness and user experience in drug delivery for pulmonary diseases. Capsules enable precise, pre-measured dosing, ensuring consistent medication delivery with each use. By isolating doses in individual capsules, the formulation is protected from environmental factors, which increases the drug's stability and shelf life. Single-use capsules also reduce the risk of cross-contamination within inhaler devices, promoting hygiene and safety. Additionally, capsule-based systems are portable, user-friendly, and compatible with various dry powder inhalers (DPIs), offering patients a convenient and reliable method of pulmonary drug administration.

In the following, preferred embodiments of the first, second and third aspect of the present disclosure are outlined in detail. It is understood that the embodiments are independent of each other and can be freely combined, if not explicitly stated or evident for the skilled person. As an example, preferred embodiments regarding the powder are generally independent of preferred embodiments for the excipient or the active agent, and these embodiments may thus be freely combined.

Pulmonary freeze-dried powder

The present disclosure relates to a spray-freeze-dried pharmaceutical powder for inhalation comprising solid particles of at least one excipient and at least one active agent, wherein the maximum of the particle size distribution of the particles is higher than 150 µm, wherein the particles are fragmented during inhalation to pulmonary particles with a median aerodynamic diameter of below 10 µm.

During inhalation, the particles are fragmented on their way from the inhaler to the lung. Only small particles can reach the pulmonary alveoli of the lungs. In the present disclosure, the fragmentation of the particles to pulmonary particles with a median aerodynamic diameter refers to the fragmentation of at least 90 %, preferably at least 95 %, still further preferably at least 99 % of the particles to break or fragment to such small particles with the given diameter.

In a preferred embodiment of present disclosure, the particles are fragmented during inhalation to pulmonary particles with a median diameter of less than 8 µm, preferably less than 5 µm.

In a preferred embodiment of present disclosure, the maximum of the particle size distribution of the solid particles is higher than 200 µm, preferably more than 300 µm, more preferably more than 350 µm and/or lower than 1000 µm, preferably less than 800 µm, more preferably less than 600 µm and most preferably less than 450 µm.

The powder of present disclosure is obtained by spray-freeze-drying of a sprayable solution wherein at least a suitable active agent and at least an excipient are dissolved.

Viscosity plays a crucial role in spray-freeze-drying (SFD) because it directly influences the droplet formation process, which is critical for producing uniformly sized particles. Moreover, viscosity affects the flow behavior through the nozzle, impacting the efficiency and reproducibility of the SFD process.

In a preferred embodiment of present disclosure, the powder is obtained by spray-freeze drying from a sprayable solution with a viscosity in the range of from 1 to about 100 mPa×s.

In another preferred embodiment of present disclosure, the powder is obtained by spay freeze-drying of an aqueous sprayable solution or a solution based on an organic solvent. The organic solvent is preferably TBA and/ or DMSO.

The powder according to present disclosure offers distinct advantages in terms of handling and flow properties, particularly because of the reduced cohesion forces and lower susceptibility to electrostatic charging, compared to a powder of finer particles.

In a preferred embodiment of present disclosure, the particles of the powder have an angle of response below 45 °.

The powder with an increased particle size compared to usual pulmonary pharmaceutical powders, as it fragments during inhalation to pulmonary effective particles. In order to exhibit the desired properties, the solid particles may have a low tendency to cohesion and at the same time be mechanically fragile enough to enable fragmentation into pulmonary particles during inhalation.

In the design of the powder according to present disclosure, particular attention was paid to fragility and cohesion tendencies of the solid particles comprised. Threshold values for each cohesion (cohesion factor $f_c$) and fragility (fragility factor $f_b$) were established to classify powders into four subgroups:

A: non-cohesive and non-fragile

B: non-cohesive and fragile

C: cohesive and non-fragile

D: cohesive and fragile

The threshold values for $f_c$ and $f_b$ were determined by data processing and calculation of the factors of different particle samples with varied parameters, after determining the particles size distribution and identifying and insolating the individual particles.

The fragility factor $f_b$ indicating a fragility tendency was determined as the factor of left shift of the particle density distribution when applying 3 kPa dispersion pressure compared to a free-fall (0 kPa) measurement. The distribution density $q_3$ is calculated based on the first derivative of the sum distribution ($Q_3$) over particle size. This leads to the equation:

$$f_b = \sum_{i=0}^{3d_n} \frac{q_{3,i}^{3kPa} - q_{3,i}^{0kPa} + \left| q_{3,i}^{3kPa} - q_{3,i}^{0kPa} \right|}{2} \cdot 10^{-2} \qquad \text{Eq. 1}$$

The cohesion factor $f_c$ indicating a cohesion tendency was defined as the difference to perfect sphericity of particles in the size range of and above singular particles in free fall. For ultrasonic nozzles, the lower size of singular particles can be approximated as 3 d, where d is the diameter of the nozzle orifice. The value for the sphericity (spht) of a particle is the ratio of the circumference of the particle to the circumference of a perfect sphere that has the same surface area as the particle. This leads to the following equation:

$$f_c = 1 - \frac{\sum_{i=3d_n}^{1000} q_{3,i}^{0kPa} \cdot spht_{3,i}}{\sum_{i=0}^{1000} q_{3,i}^{0kPa}} \qquad \text{Eq. 2}$$

In a preferred embodiment of present disclosure, the powder has a fragility factor $f_b \geq 0.25$ and a cohesion factor $f_c \leq 0.17$.

In a preferred embodiment of present disclosure, the powder has a bulk density in a range between 0.01 g/ml to 0.25 g/ml. Preferably, the bulk density is below 0.15 g/ml, preferably below 0.10 g/ml, more preferably below 0.070 g/ml, even more preferably below 0.050 g/ml, and most preferably below 0.030 g/ml.

In another preferred embodiment of present disclosure, the powder has a bulk density of 0.02, 0.025, 0.03, 0.04 or 0.05 g/ml.

Low bulk density powders are less likely to settle and aggregate within inhalation devices, resulting in more consistent dosing. This is particularly beneficial for dry powder inhalers (DPIs), where free-flowing particles are essential for accurate and reliable dose delivery. Low-density powders are also less prone to clumping due to reduced gravitational force between particles, maintaining the stability and homogeneity of the formulation over time.

In a preferred embodiment of the powder of present disclosure the particles undergo fragmentation by inhalation to pulmonary particles without the need of a further modified active inhalation device that triggers fragmentation.

In a preferred embodiment, the powder comprises solid particles with a calculated geometric density that is in a range between 0.03 and 0.25 g/ml, preferably between 0.04 and 0.15 g/ml, more preferably between 0.05 and 0.1 g/ml, most preferably between 0.04 and 0.08 g/ml.

In another preferred embodiment of present disclosure, the powder comprises solid particles with a calculated geometric density in a range between 0.03 and 0.07 g/ml, preferably wherein the calculated geometric density is 0.05 g/ml.

In another preferred embodiment of present disclosure, the powder comprises solid particles with a calculated geometric density in a range between 0.07 and 0.12 g/ml, preferably wherein the calculated geometric density is 0.1 g/ml.

In another preferred embodiment of present disclosure, the powder comprises solid particles with a calculated geometric density in a range between 0.12 and 0.18 g/ml, preferably wherein the calculated geometric density is 0.15 g/ml.

In another preferred embodiment of present disclosure, the powder comprises solid particles with a calculated geometric density in a range between 0.18 and 0.25 g/ml, preferably wherein the calculated geometric density is 0.2 g/ml.

In a preferred embodiment the powder of present disclosure has a fine particle fraction (FPF) of the pulmonary particles after fragmentation of more than 30 %, preferably more than 40 %, even more preferably more than 50 %, most preferably more than 60 %.

The fine particle fraction (FPF) is a critical parameter in the administration of pulmonary powders, as it indicates the proportion of aerosolized particles that are small enough to penetrate deep into the lungs, specifically targeting the alveolar region. A higher fine particle fraction suggests that a greater percentage of the delivered dose of an active agent is likely to reach the intended site of action, enhancing the therapeutic efficacy of the medication. Conversely, a low FPF may result in suboptimal lung deposition and reduced treatment effectiveness. Therefore, optimizing the fine particle fraction in the formulation and delivery of pulmonary powders is essential for achieving the desired outcomes in respiratory therapies.

Excipients

The powder of the present disclosure comprises at least one excipient. The excipient is characterised by biocompatibility allowing safe and effective use in pulmonary drug delivery systems. It can be further advantageous to use excipients with further beneficial properties. Hygroscopic or moisture-resistant properties are beneficial to extend the shelf-life by maintaining the stability of the powder. Furthermore, excipients that enable facilitated aerolization or comprise stabilizing properties for active agents may be selected for beneficial reasons.

In a preferred embodiment of the present disclosure, at least one excipient is selected from the group consisting of gelatine, maltodextrin (glucidex), trehalose, lactose, mannitol or mixtures thereof. Preferably, the excipient is mannitol. Suitable excipients may also be hydroxypropyl cellulose, PVA, PEG, PVP, or other biocompatible excipients.

In a preferred embodiment of the present disclosure, the excipient is present in the powder in an amount of at least 80 % w/w, preferably at least 85 % w/w, and most preferably at least 88 % w/w, based on the total weight of the powder.

In a particular preferred embodiment of the present disclosure, the excipient is mannitol, based on 5 % solid content.

In a particular preferred embodiment of the present disclosure, the excipient is mannitol with 88 % w/w content in total based on the total weight of the powder.

In a particular preferred embodiment of the present disclosure, the excipient is mannitol with 4.4 % w/w content in the spraying solution prior to spray-freeze drying resulting in the powder of present disclosure.

Active agent

The powder of the present disclosure comprises at least one active agent.

Suitable active agents typically include bronchodilators, corticosteroids, antibiotics, mucolytics, antivirals, antifungals, anti-inflammatory agents, pain management agent and vaccines or biologics designed for pulmonary delivery. Active agents in pulmonary administration must meet specific criteria to ensure safety, efficacy, and stability in the lungs.

Active agents for pulmonary administration need to resist degradation during processing, storage, and in the humid environment of the lungs. The solubility of the active agent impacts its absorption and duration of action.

In a preferred embodiment of the present disclosure, the powder comprises at least one suitable active agent with a content in total of less than 20 % w/w, preferably less than 15 % w/w, more preferably equal or less than 12 % w/w, based on the total weight of the powder.

The content in the powder is dependent on the previous content of the active agent in the spraying solution prior to spray-freeze drying.

The content of the active agent in the spraying solution is between 0.3 and 1% w/w, when the total solid content of the final spraying solution comprising the active agent and the excipient is equal or lower than 25 %, preferably lower than 10 % and more preferably equal or lower than 5 %. In particular, the content of the active agent in the spraying solution is preferably 0.6 % w/w at a total solid content in the spraying solution of 5 %.

In a preferred embodiment of present disclosure, the active agent is selected from the group consisting of bronchodilators, corticosteroids, antibiotics, mucolytics, antivirals, antifungals, anti-inflammatory agents and vaccines or biologics designed for pulmonary delivery.

In another preferred embodiment of present disclosure, the suitable active agent may be selected from Salbutamol, Salmeterol, Formoterol, Terbutaline, Ipratropium bromide, Tiotropium bromide, Budesonide, Fluticasone, Beclomethasone, Mometasone, Ciclesonide, Tobramycin, Aztreonam, Ciprofloxacin, Colistin, Levofloxacin, Acetylcysteine, Dornase alfa, Zanamivir, Ribavirin, Amphotericin B, Voriconazole, Cromolyn sodium, Montelukast, Nedocromil sodium, Palivizumab, Dupilumab, Adalimumab, Infliximab, Influenza vaccine, COVID-19 vaccines, inhalable insulin (Afrezza), Fentanyl, Nitric oxide, Treprostinil and the like.

In a further preferred embodiment of present disclosure, the active agent is selected from Salbutamol, Salmeterol, Formoterol, Terbutaline, Ipratropium bromide, Tiotropium bromide.

In a particular preferred embodiment of present disclosure, the active agent is Salbutamol.

In another preferred embodiment of present disclosure, the active agent may be insoluble in water.

In a preferred embodiment of present disclosure, the insoluble active agent may be Aprepitant.

Treatment of diseases

The powder of the present application may be used for the treatment or alleviation of diseases or conditions in an animal, preferably a mammal, further preferably a human. The diseases that can be treated with the powder according to the present disclosure are pulmonary related diseases or conditions, based on the fragmentation to pulmonary particles of the powder comprising solid particles.

In a further aspect, the present disclosure relates to the powder for use in the treatment of pulmonary diseases.

Pulmonary diseases are a group of medical conditions that affect the lungs and respiratory system, impairing the body's ability to breathe effectively and oxygenate the blood. These diseases can impact the airways, lung tissue, blood vessels, or the pleura (the lining surrounding the lungs), leading to symptoms such as coughing, shortness of breath, wheezing, chest pain, and reduced lung function. Pulmonary diseases include both acute conditions and chronic conditions. Causes vary widely, including infections, environmental factors (like smoking and air pollution), genetic predispositions, and autoimmune reactions.

The powder of the present disclosure may be used e.g. for the treatment of Asthma, Chronic Obstructive Pulmonary Disease (COPD), Pulmonary Fibrosis, Cystic Fibrosis, Pulmonary Hypertension, Bronchiectasis, Pneumonia, Tuberculosis (TB), Acute Respiratory Distress Syndrome (ARDS), Lung Cancer, Pulmonary Embolism (PE), Sarcoidosis, Interstitial Lung Disease (ILD), Alpha-1 Antitrypsin Deficiency, Bronchiolitis Obliterans, Hypersensitivity Pneumonitis, Pleural Effusion, Asbestosis, Respiratory Syncytial Virus (RSV) Infection and the like.

Pulmonary particles are administered into the deep lungs primarily through inhalation devices, designed to deliver medications directly to the lower respiratory tract. The goal is to achieve effective particle deposition in the alveolar region, which requires particles of an optimal size to avoid settling in the upper airways and to reach the deep lung tissue.

In a preferred embodiment, the powder of present disclosure for use in a treatment is dispersed by an active type of dry powder inhaler (DPI).

In another preferred embodiment, the powder of present disclosure for use in a treatment is dispersed by a passive type of dry powder inhaler (DPI).

Passive types of dry powder inhalers solely on the patient's inhalation effort to disperse and deliver the powdered medication to the lungs. Unlike active DPIs, passive DPIs do not contain any mechanical or propellant-driven mechanisms to release the drug.

In a further aspect, the present disclosure relates to a capsule suitable for provision in an inhalation system comprising the powder according to present disclosure.

When selecting materials for capsules designed for use in dry powder inhalers (DPIs) containing pulmonary powders, several factors must be considered, including biocompatibility, compatibility with the drug, stability, and performance during inhalation.

Suitable materials for capsules designed for dry powder inhalers (DPIs) containing pulmonary powders include gelatin, hydroxypropyl methylcellulose (HPMC), pullulan, and starch-based materials. Gelatin is widely used for its biocompatibility and ease of manufacture, while HPMC and pullulan offer excellent moisture resistance and are suitable for vegetarian formulations. Modified starch can also provide moisture protection and stability.

Definitions

In the following, a number of definitions is given for terms as they are used in the context of the present disclosure.

Where the term "solid particles" is used in the present description or claims, the particles result from a sprayed droplet during freeze-drying. Particles that resemble an agglomerate of several small individual particles and are held together by interactions between these smaller components are explicitly excluded.

The term "maximum of the particle size distribution" in the description or the claims is to be understood as the absolute maximum over the whole particle size distribution curve of particle count vs. particle size determined by dynamic image analysis (DIA).

The term "aerodynamic diameter" refers to a parameter that is influenced by density (geometric particle density $\rho_{geo}^{pa}$ , density reference particle $\rho^{ref}$), geometric diameter ($d_g$), shape and surface roughness (shape factor $\lambda$) of the particles contained in the powder. The values for an aerodynamic diameter are estimated as follows:

$$d_a = d_g \cdot \sqrt{\frac{\rho_{geo}^{pa}}{\lambda \cdot \rho^{ref}}} \qquad \text{Eq. 3}$$

The term "median aerodynamic diameter" is the value of the aerodynamic diameter for which 50 % of a quantity in a given aerosol is associated with particles smaller than the median aerodynamic diameter, and 50 % of the quantity is associated with particles larger than the median aerodynamic diameter. It simplifies the true distribution of aerodynamic diameters of a given aerosol as a single value. The "mass median aerodynamic diameter" (MMAD) was calculated as described in the USP31 <601> by plotting the cumulative percentage of mass found on each stage against the less than stated aerodynamic of the respective stage and determining the mass median aerodynamic particle size by fitting a linear model and calculating AD at 50 % of the recovered mass.

The terms "active agent with a content" or "excipient with a content" in the description or the claims refers to mass percent of the absolute mass of the contained active agent or excipient in relation to the absolute mass of the measured spray freeze-dried powder according to the present application.

The term "$x_{50}$" in particle size analysis refers to the median particle diameter, meaning that the number of particles in a sample having a particle size smaller than $x_{50}$ is 50 %, and 50 % have a larger median particle. It is often used to characterize the average particle size in a distribution and is also called the $D_{50}$ value. Due to their porosity, the particles are not characterised by their mass per particle but by the number of particles in the respective size range and should therefore be seen as distinct from a mass-dependent diameter (e.g. MMAD).

The term "solid content" refers to the ratio of the content (mass) of all solid components (e.g. active agent and excipient) that are solved or dispersed in the spraying solution (mass or volume, depending on the solvent used) prior to spray-freeze-drying the solution to form the solid particles.

The term "calculated geometric density" refers to the value calculated from the solid content of the solution prior to spraying, assuming complete evaporation of the solvent and no collapse of the particles that are obtained after spray-freeze drying.

The term "sphericity" refers to the calculated ratio of the circumference of the particle to the circumference of a perfect sphere that has the same surface area as the particle.

Where the term "bulk density" is used, it is referred to the density that is determined by measuring the volume and the corresponding mass.

Where the term "fine particle fraction" (FPF) is used, it is referred to the proportion of particles that can reach deep into the lungs and typically have a particle size of less than or equal to 5 $\mu$m. In relation to experiments on lung deposition using the next generation impactor (NGI), the fine particle fraction is generally calculated from the mass fractions of the particles that are deposited at stage 2 to stage 7.

The term "passive type of a powder inhaler" refers to dry powder inhalers (DPI) in which the aerosol is formed and the medication is released solely by the patient's breath. No additional energy source or mechanism of the inhaler itself is used to nebulise or fragment the powder.

The term "active type of a powder inhaler" refers to a dry powder inhaler in which the release and distribution of the medication is supported not only by the patient's breathing force, but also by an external energy source. This energy source can be a motorised mechanism, compressed air or an electrical device integrated in the inhaler that activates and disperses the powder to transport it into the airways.

Where an indefinite or definite article is used when referring to a singular noun, e.g. "a", "an" or "the", this includes a plural of that noun unless specifically stated otherwise.

The term "at least one" refers to a number of 1 or more representations of the following term, in particular active agent or excipient. The representations of the respective material, if present more than once, i.e., 2, 3, 4, or more times, may be independently selected from each other within the definition of the respective material, particularly preferred 1 or 2 times.

EXPERIMENTAL SECTION

In the following, the present disclosure is illustrated in more detail with reference to the following examples. The following examples are set forth to assist in understanding the disclosure and should not be used as specifically limiting the disclosure described and claimed herein. Such variations of the disclosure, including the substitution of all equivalents now known or later developed, would be within the purview of those skilled in the art, and changes in formulation or changes in experimental design, are to be considered to fall within the scope of the disclosure incorporated herein.

Methods for Characterization

The bulk density is determined by measuring the powder volume and corresponding mass. Flowability experiments are conducted analogous to the angle of repose measurement described in Chapter 2.9.36 of the European Pharmacopoeia.

The viscosity of test solutions in water, DMSO, or TBA were measured using a rotational Rheometer MCR92 (Anton Paar, Graz, Austria). The viscosimeter was filled with 20 ml of the test solution into a concentric cylinder setup (CC27 Unit) and measurements were run under a constant shear rate of 20/s or 50/s at 25 °C. Measurement diagrams with 20 points were created to determine the viscosity.

For the determination of median particle size, sphericity (roundness), and geometric particle size distribution, dynamic image analysis (DIA) is performed (Retsch Camsizer, X-Dry Module). In this process, the powder is filmed in free fall by two camera systems, and the particle size distribution is created based on the captured images. A minimum of 30,000 particles were measured for each sample using the high-resolution camera (for particle sizes above 0.8 $\mu$m) and 1,000,000 particles using the base camera (particle sizes up to a maximum of 3 mm). The sphericity of the particles is calculated based on their perimeter.

Mechanical stability testing is performed by applying dispersing pressure to the DIA. Prior to reaching the camera, the particles are accelerated with pressure, causing mechanically unstable formulations to break, resulting in a correspondingly smaller median particle size and decreased sphericity. A fragility screening is conducted using increasing pressures to obtain a pre-selection of suitable particles. Mechanically stable formulations, as well as strongly agglomerating samples, were excluded from further measurements.

The non-excluded suitable particles undergo a test procedure for aerosol classification using the Next Generation Impactor (NGI). The Next Generation Impactor (NGI) consists of a mouthpiece adapter, a preseparator, seven cups, or stages, and a filter, also referred to as micro-orifice collector (MOC). The instrument is described by the USP31 <601> as "Apparatus 5". Each part is serving a specific function:

- Induction Port (IP): Acts as a connector between the inhaler and the preseparator

- Preseparator (Pres): An additional stage that retains larger particles and allows smaller particles to pass through to avoid overloading of Stage 1

- Stage 1 (S1): Also retains particles too large to enter the lungs, similar to the preseparator

- Stages 2-7 (S2-S7): These cups are sized incrementally to capture particles of varying sizes, with larger particles collected in the earlier (smaller numbered) cups and smaller ones in the later ones.

- Micro-orifice collector (MOC): terminal collector cup for the smallest powder particles.

Together, these cups enable the NGI to accurately determine the particle size distribution of an inhalation powder by segregating and capturing particles based on their sizes.

The formulations were tested for administration with the Lupihaler and therefore previously filled into hydroxypropyl methylcellulose (HPMC) capsules before actual application according to usual practice. The NGI was used in conjunction with two vacuum pumps. The inhaler was connected to the inlet of the impactor with a suitable mouthpiece. Prior to the experiments, 15 mL of the dissolution medium were added to the pre-separator, and each cup (S1-MOC) was coated with a 1 % glycerin-methanol solution (w/v) to prevent rebound effects after particle impact. For the quantification of the active ingredient in the various stages (cups - MOC), the particles were dissolved in water : methanol (50 : 50 v/v (%)). For each experiment, an actuation volume of 4 liters in combination with a flow rate of 100 L/min was used for the Lupihaler DPI, which closely corresponds to USP conditions (USP31 <601>) with a pressure drop of 4 kPa.

Active ingredient quantification is determined by the active ingredient deposit in various NGI stages using high-performance liquid analysis (HPLC) with an RP18 column. The photodiode array detector was set at 275 nm. The mobile phase consisting of a phosphate buffer pH 3/methanol (80:20 v/v (%)) was set to a flow rate of 1.4 mL/min, and the active ingredient was quantified at a column temperature of 40 °C. To calculate the limit of detection (LOD) and limit of quantification (LOQ), the values of the y-intercepts and the slope of the calibration curve were used. The LOD and LOQ were determined to be 0.36 $\mu$g/mL and 1.08 $\mu$g/mL, respectively.

The Fluorescein samples were pipetted into 96-well plates and excited at a wavelength of 490 nm while the emission was measured at 520 nm using an EnSpire Multimode Plate Reader. Quantification of the test substance was performed at a temperature of 25 °C.

To enable correlation between geometric and aerodynamic radius, the stages of the NGI were equipped with conductive adhesive tape, and two capsules (each containing 10 mg) were measured using the NGI. The adhesive tapes were then sputtered with gold (GaLa Instruments, 15 mV, 2 min) along with the particles or particle fragments adhering to them to increase conductivity, and their size was determined using scanning electron microscopy (Hitachi SU3500) at 5 kV and 300x magnification.

Modified mouthpieces for inhaler were manufactured by 3D printing. The component was designed using Autodesk Fusion 360. For printing the model, a masked stereolithography printer was used (Prusa SL1S). After printing, post-processing steps were carried out, including cleaning the printed surface with isopropanol and compressed air to remove any excess resin from the component's cavities and surfaces. Subsequent surface curing was performed at 25 °C under UV light. Due to the complex geometry of the prints, curing was repeated several times from different angles to ensure complete resin curing.

Example 1: General method for producing the spray-freeze-dried inhalable powder

According to the solid concentrations, the masses of excipient and active ingredient are filled up with water to 100 g, dissolved, and filtered through a 5 $\mu$m cellulose acetate filter. The spray solutions are passed through a droplet generator (FMP, Erlangen) with an outlet nozzle of 100 $\mu$m at an overpressure of 170 kPa, with the generated droplets freezing in free fall in a chamber cooled to -120 °C. The powder was collected at the bottom of the tower and then transferred to a freeze dryer (Alpha 1-4 LSC Plus, Martin Christ). Drying was carried out for 48 hours at 0.1 mbar and 20 °C.

Alternatively, drying of the spray frozen particles can be performed under atmospheric conditions. To remove the solvent by atmospheric sublimation, the particles are then dried in the same chamber.

Nitrogen or dry air can be used as the drying gas, preferably in a temperature range of -20 to -5 °C for aqueous solutions, whereby the drying gas passes through the ice particles until the particles are dry. The dryness can be determined by means of the residual solvent content in the final product or in the exhaust gas. The process is typically stopped at approx. 20 ppm.

Example 2: Viscosity measurements of various excipients in water

The viscosity of various excipients was tested to analyze the spray-ability during the spray-freeze drying process through FMP nozzles of a droplet generator (see Tables 1 and 2).

Table 1 Different excipient compositions and their resulting viscosity in water.

| Solid content [%] | Viscosity [mPa·S] | Angle of Repose [°] |
|---|---|---|
| Maltodextrin | | |
| 5 % | 1.22283± 0.001 | 55 |
| 10 % | 1.56159± 0.001 | 48 |
| 15 % | 2.10584 ±0.003 | 44 |
| 20 % | 2.55395 ±0.002 | 37 |
| 30 % | 4.26214±0.005 | 34 |
| 44 % | 6.53888±0.004 | 30 |
| HPC (SSL) | | |
| 5 % | 6.00693±0.010 | 66 |
| 8 % | 12.9537±0.004 | 62 |
| 10 % | 20.4936±0.030 | 55 |
| 13 % | 41.4784±0.030 | 44 |
| Mannitol | | |
| 5 % | 1.13296 ±0.001 | 40 |
| 10 % | 1.31717± 0.001 | 37 |
| 15 % | 1.46901± 0.002 | 35 |
| 17 % | 1.54544 ± 0.002 | 33 |
| 20 % | 1.65831 ± 0.001 | 30 |
| PVP (KOLLIDON®17) | | |
| 5 % | 1.39728±0.001 | 53 |
| 10 % | 1.9613±0.002 | 39 |
| 15 % | 3.63167±0.002 | 36 |
| 20 % | 3.95396±0.003 | 34 |
| 34 % | 7.98471±0.003 | 28 |
| PVP (KOLLIDON®25) | | |
| 5 % | 1.90252±0.001 | 46 |
| 10 % | 3.62791±0.001 | 44 |
| 15 % | 6.07313±0.003 | 34 |
| 20 % | 9.90467±0.002 | 30 |

Table 2 Viscosity measurements of hydroxypropyl cellulose types in water, tert-butanol (TBA), and dimethyl sulfoxide (DMSO), measurements were run n=1

| | viscosity (mPas) | | |
|---|---|---|---|
| solid content (w/v) | water | TBA | DMSO |
| HPC-UL | | | |
| 5 % | 2.7 | 21.5 | 5.5 |
| 10 % | 7 | 74.3 | 13.8 |
| HPC-SSL | | | |
| 5 % | 5.5 | 47.5 | 10.8 |
| 10 % | 19.5 | 246.6 | 31.1 |

Results on aqueous solutions and their respective viscosity providing sufficiently low viscosity for obtaining SFD particles.

All spray solutions of Tables 1 and 2, except of 10 % HPC-SSL in TBA, were sprayable with a FMP nozzle using nozzle orifices ranging from 50 to 200 µm and led to free-flowing, spherical particles with a maximum of the particle size distribution below 1000 µm (measured via DIA).

Compared to two-way nozzles, the limitation by the spray solution viscosity occurs at a surprisingly low level. While current literature mentions that liquid formulations with a maximum viscosity of 350 mPa×s can be sprayed within conventional nozzles i.e., pressure and rotary atomizers; however, state-of-the-art monodisperse nozzles can spray fluids with viscosity up to 500 mPa×s. (S. M. Mayramnegari et al., Journal of Food Engineering (338), 2023). However, piezo-ultrasound-driven, e.g. the FMP nozzle and as most relevant here, were surprisingly found to be much more limited to higher viscosities. Accordingly, SFD solutions were found to be sprayable at viscosity ranges of around 1 to around 100 mPa×s of the spray solutions, mainly impacted by the orifice diameter, varying between 10 and 300 μm. The spray-ability was discovered to be independent from solvent type and excipient type and concentration. A solution is found to be sprayable when clogging of the nozzle or coalescence of the spray droplet at the nozzle does not occur.

Example 3: Bulk densities in dependency of the solid content

Table 3 Different particle compositions and their resulting densities in dependency of the solid content and further examination NGI experiments.

|  | Solid content (w/w) | Bulk density (g/ml) | Calculated geometric density (g/ml) |
|---|---|---|---|
| Mannitol | 3 | 0.021 | 0.03 |
|  | 5 | 0.026 | 0.05 |
|  | 10 | 0.057 | 0.10 |
|  | 15 | 0.089 | 0.15 |
|  | 20 | 0.122 | 0.20 |
| Lactose | 3 |  | 0.03 |
|  | 4 |  | 0.04 |
|  | 5 | 0.031 | 0.05 |
| Maltodextrin | 5 | 0.028 | 0.05 |
| Trehalose | 5 |  | 0.05 |
| PEG | 5 | 0.031 | 0.05 |
|  | 10 | 0.060 | 0.10 |
|  | 15 | 0.103 | 0.15 |
| Gelatine | 5 | 0.024 | 0.05 |
| PVP:Mannitol | 1.5:1.5 | 0.023 | 0.03 |
|  | 2.5:2.5 | 0.057 | 0.05 |
|  | 5:5 | 0.081 | 0.10 |
|  | 7.5:7.5 | 0.119 | 0.15 |
|  | 10:10 | 0.159 | 0.20 |
| PVA | 5 | 0.037 | 0.05 |
| Salbutamol | 5 |  | 0.05 |
| Mannitol:Salbutamol | 2.4:0.6 | 0.016 | 0.03 |
|  | 3.4:0.6 | 0.022 | 0.04 |
|  | 4.4:0.6 | 0.026 | 0.05 |
|  | 2.5:2.5 | 0.030 | 0.05 |
|  | 3.8:1.2 | 0.032 | 0.05 |
|  | 4.5:0.5 | 0.029 | 0.05 |
| Mannitol:Aprepitant | 5:5 |  | 0.10 |
| Mannitol:Tobramycin | 2.5:2.5 | 0.031 | 0.05 |
| Tobramycin | 5 | 0.025 | 0.05 |
|  | 7 | 0.039 | 0.07 |

(continued)

| | Solid content (w/w) | Bulk density (g/ml) | Calculated geometric density (g/ml) |
|---|---|---|---|
| Mannitol:Sumatript | 3.8:1.2 | 0.028 | 0.05 |

Example 4: NGI experiments for different excipients

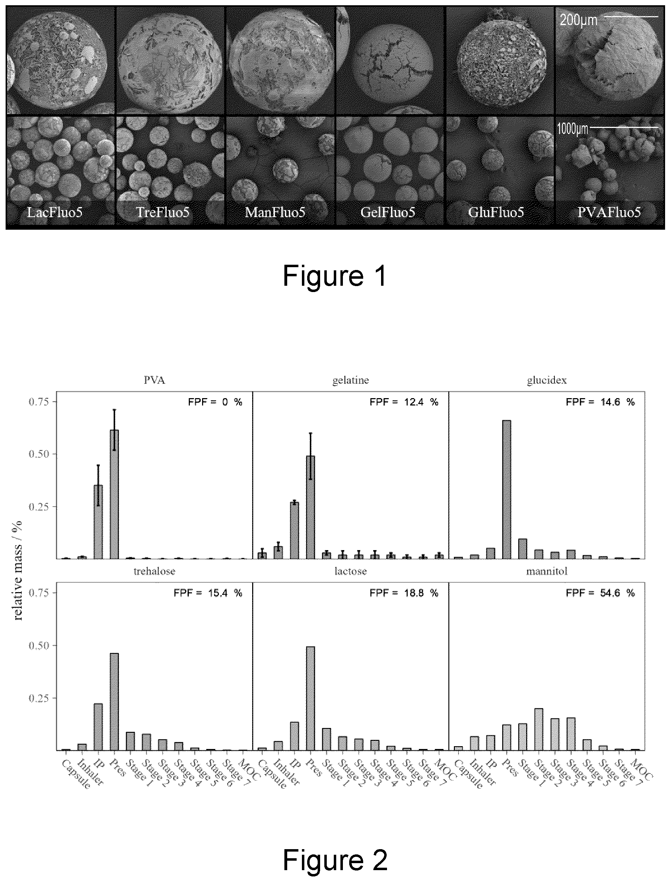

In aim to investigate the aerosolization properties of various spray-freeze-dried formulations, NGI (next generation impactor) experiments were conducted with commonly utilized excipients for pulmonary application and the placebo fluorescein. Different excipients exhibit a wide range of properties in respect to mechanical stability. Five formulations containing different excipients with a 5 % solid content were prepared. The particle size of the produced spray-freeze-dried powders was analysed by SEM image views (Figure 1). All prepared powders were obtained in particles sizes above 200 $\mu$m (median diameter approximately 375 $\mu$m). Particles of this size would not fall under the classification of pulmonary particles and would therefore not be considered to be suitable for pulmonary administration. It was surprisingly found that the results for lung deposition were very promising with fine particle fractions above 10 % (EF= emitted fraction; TD = total dose) for all formulation except from PVA polymer-based particles. Particle formulations containing gelatine, maltodextrine (glucidex), lactose and trehalose surprisingly resulted in FPF values reached by common commercial products for pulmonary administration (Table 4; Figure 2). Even more surprisingly, the mannitol-based particles overachieved this value by more than threefold exhibiting very high FPF values about approximately 55 % (Table 4; Figure 2). The experiment proofed good properties for pulmonary administration of large particles (>200 $\mu$m diameter) according to present application, that fragment to smaller particles (1-5 $\mu$m mass median aerodynamic diameter (MMAD)) upon inhalation.

Table 4 Lung deposition (NGI) for various excipients at 5 % solid content.

| Excipient | Solid content [%] | FPFEF [%] | FPFTD [%] | MMAD [$\mu$m] |
|---|---|---|---|---|
| PVA | 5 | 0.0 | 0.0 | - |
| Gelatine | 5 | 12.4 | 11.4 | 3.06 |
| Glucidex | 5 | 14.6 | 14.2 | 4.23 |
| Tehalose | 5 | 15.4 | 14.8 | 4.22 |
| Lactose | 5 | 18.8 | 17.8 | 4.01 |
| Mannitol | 5 | 54.6 | 49.9 | 3.15 |

Example 5: Solid content variation of the formulation

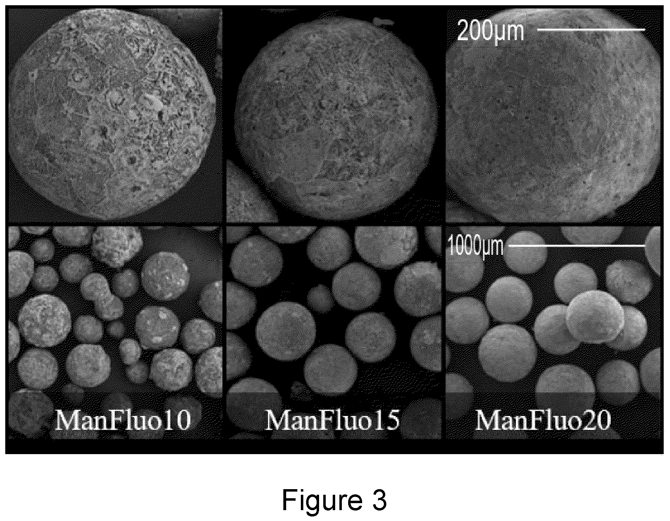

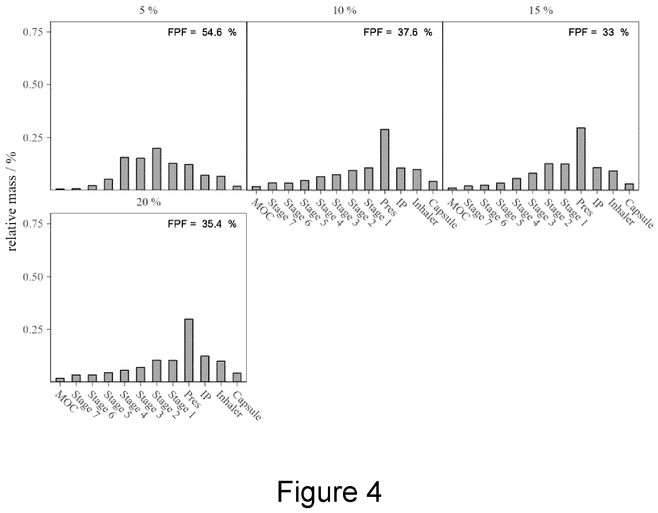

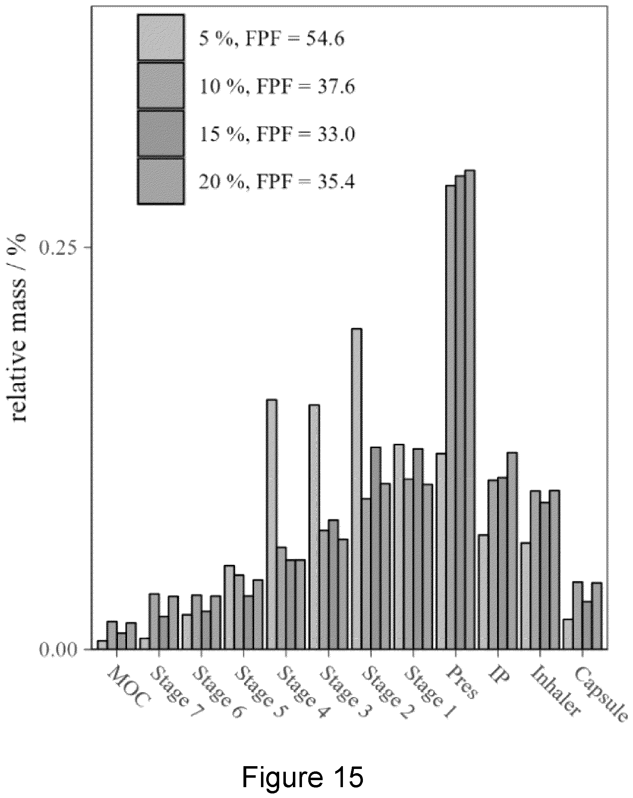

The effect on the FPF in different solid content formulations of spray-freeze-dried particles was tested in formulations containing 5, 10, 15, and 20 % solid content of mannitol. The obtained particles were analyzed by SEM image view (Figure 3) and NGI experiments for lung deposition properties (Figures 4 and 15). It was found that the lung permeability decreases with increasing solid content of the formulation.

Table 5 Lung deposition for mannitol-based particles at different excipient concentrations.

| Excipient | Placebo/ active agent | Conc. Placebo/ active agent [%] | Solid content [%] | FPFEF [%] | FPFTD [%] | MMAD [$\mu$m] |
|---|---|---|---|---|---|---|
| Mannitol | Fluorescein | 0.03 | 5 | 54.6 | 49.9 | 3.15 |
| Mannitol | Fluorescein | 0.03 | 10 | 37.6 | 32.3 | 3.07 |
| Mannitol | Fluorescein | 0.03 | 15 | 33.0 | 29.0 | 3.81 |
| Mannitol | Fluorescein | 0.03 | 20 | 35.4 | 30.5 | 3.29 |
| Mannitol | Aprepritant | 2.5 | 5 | 40.2 | 37.2 | 2.95 |

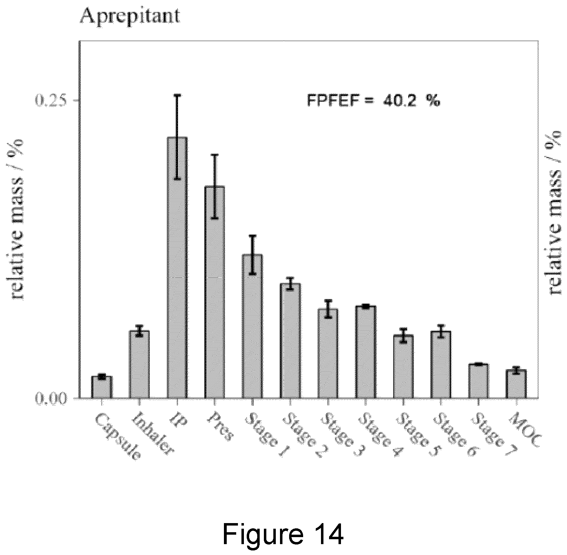

However, all of the formulations still reached FPF values above 30 % (Figure 14 and 15). The powders with a higher solid content appear to accumulate in the pre-separator. For lower concentrated mannitol formulations, the relative masses are increased on stages 2-5, whereas higher concentrations of mannitol result in slight elevated values on stages 6, 7 and MOC.

Example 6: Active agent concentration of the formulation

Salbutamol concentration in varying total solid content of mannitol-based particles was investigated in their effect on the FPF values. The formulation of the salbutamol spray-freeze-dried (SFD) powders was designed in a way that enables the same salbutamol dose in each capsule, regardless of the varying total solid content. 170 $\mu$l SFD powder (the volume of one capsule) in a close packing sphere therefore results in 1 mg of salbutamol.

**Table 6** Formulation of spray freeze-dried powders containing salbutamol and mannitol

|  | In spraying solution | | In powder | |
|---|---|---|---|---|
| Solid content [%] | Salbutamol [w%] | Mannitol [w%] | Salbutamol [w%] | Mannitol [w%] |
| 3 | 0.6 | 2.4 | 20 | 80 |
| 4 | 0.6 | 3.4 | 15 | 85 |
| 5 | 0.6 | 4.4 | 12 | 88 |

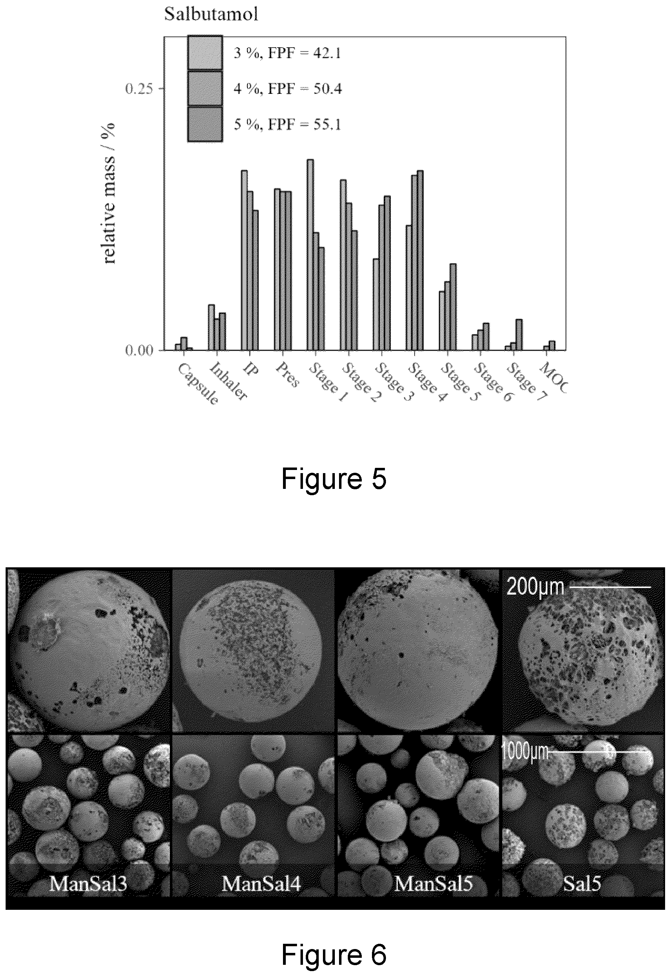

The inclusion of 0.6 % salbutamol in the spray solution of a mannitol-based SFD powder with 5 % solid content resulted in a FPF of 55.1 % (Table 7, Figure 5). SEM images of all solid content formulations are shown in Figure 6.

**Table 7** Results of the NGI experiments and determination of aerodynamic diameter (MMAD) of the formulations presented in table 6

| Solid content [%] | FPFEF [%] | FPFTD [%] | MMAD [$\mu$m] | X50 [$\mu$m] |
|---|---|---|---|---|
| 3 | 42.1 | 40.1 | 3.83 | 411 |
| 4 | 50.4 | 48.3 | 2.72 | 408 |
| 5 | 55.1 | 53.0 | 2.39 | 405 |

For comparison the experiment was repeated using 2.5 % aprepitant nanocrystals and 2.5 % mannitol (total solid content: 5 %). Aprepitant is commonly used to treat nausea in patients receiving chemotherapy. Furthermore, aprepitant nanocrystals are insoluble, thus prepared in a suspension instead of a solution that is subsequently spray-freeze dried.

*Preparation of the 5 % Aprepitant nanocrystal suspension:*

A sprayable nanosuspension was produced using a ball mill. Two grinding drums were each filled with 15 ml of zinc oxide beads and 8 ml of grinding material. The grinding material consists of hydroxypropylcellulose (HPC) and sodium dodecyl sulphate (SDS) as stabilizing agents for the nanosuspension and the active pharmaceutical ingredient.

**Grinding Material**

| mHPC (g) | mSDS (g) | mAprepitant (g) | Water (ml) |
|---|---|---|---|
| 0.12 | 0.16 | 2.0 | 8.0 |

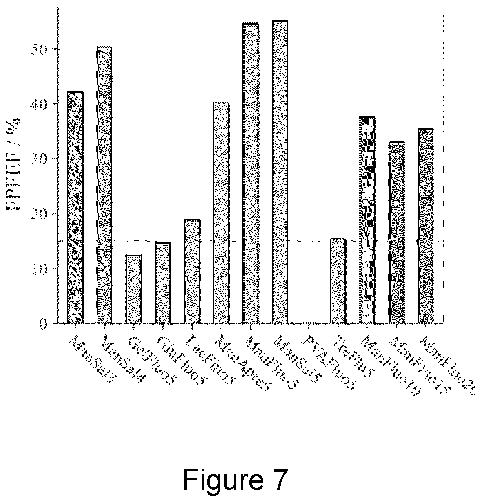

The mixture was milled for 4 hours at 30 Hz. The suspension was then mixed with mannitol solutions using a magnetic stirrer to achieve a total 5 % solid content (2.5:2.5). The final suspension was filtered through a 100 $\mu$m filter.
The formulation led to an FPF similar to those of 10 % solid content mannitol SFD powder of 40 %, with a decreasing share of the drug found in increasing stage depth (Figure 14). It was shown that particles according to present invention can also be prepared from suspensions with insoluble active agent. An overview over the NGI experiments with resulting FPF values is presented in Figure 7.
Reducing the solid content while maintaining a concentration of 0.6 % salbutamol led to a decrease in FPF and an increase in fragility.

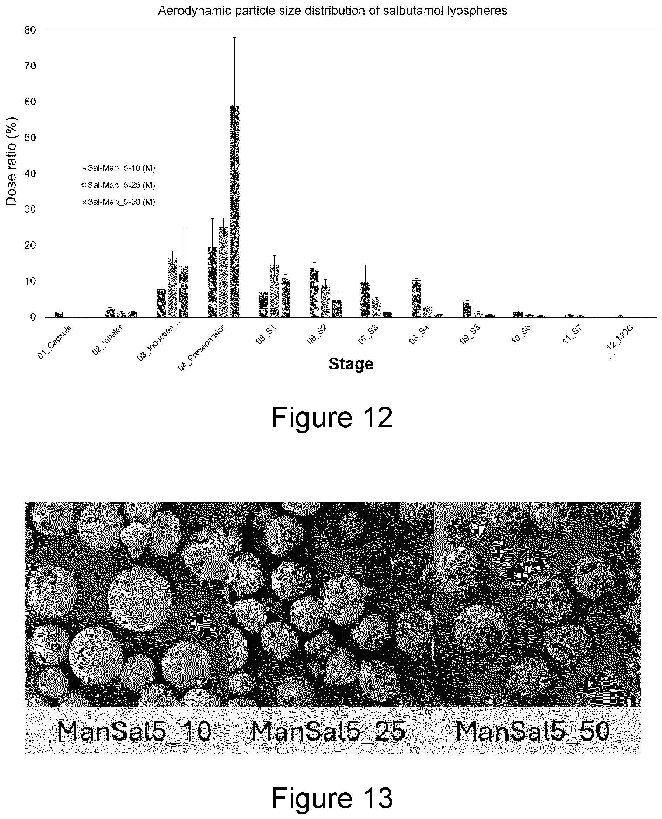

In further experiments with particles of mannitol as excipient and salbutamol as active agent at a solid content of 5 % in total, it can be concluded that the proportion of respirable particles is adjustable via the proportion of active agent to excipient (Table. 8, Figure 12 and SEM images Figure 13). This offers the advantage that the amount of active agent that should effectively reach the deeper lungs can be adjusted relative to the excipient proportion.

**Table 8** Lung deposition of particle fractions (PF) dependant on the proportion of active agent (salbutamol) to excipient (mannitol) in particles with 5 % solid content in total

| Salbutamol content [%] | PF Capsule-Stage 1 [%] | FPF Stage 2 - Stage 6 [%] | X50 [$\mu$m] |
|---|---|---|---|
| 10 | 38.13 | 40.37 | 282 |
| 25 | 57.95 | 19.83 | n.d.* |
| 50 | 85.69 | 8.82 | 349 |
| *n.d. = not determined | | | |

Example 7: Additional use of active inlays in inhalation devices

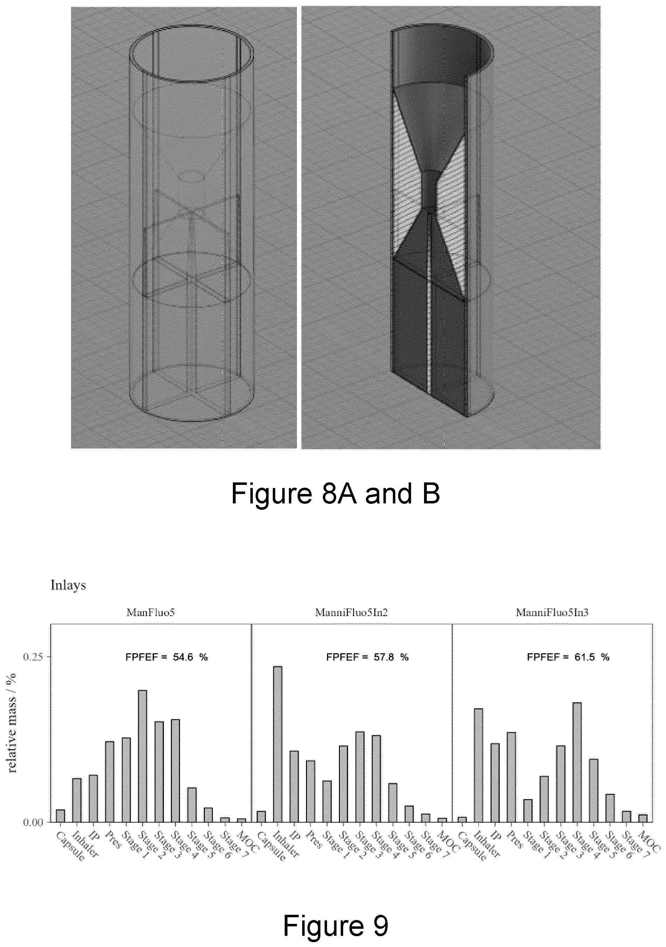

The lung permeability of spray-freeze-dried powders of the present disclosure was further investigated with modified inhalation device inlays. 3D printed inlays (Figure 8A and 7B) that can be inserted into the mouthpiece of commercially available inhalation devices were used in this example. These types of inlays allow for a reduction of the aerodynamic diameter and thus targeted modulation of the particle size. Inlay one exhibits stages reaching into the mouthpiece, whereas inlay two exhibits sand-clock shape to reach this reduction. Spray-freeze-dried powders of present disclosure achieved surprisingly high FPF values of 61.5 % (Figure 9).

Example 8: Breaking behavior

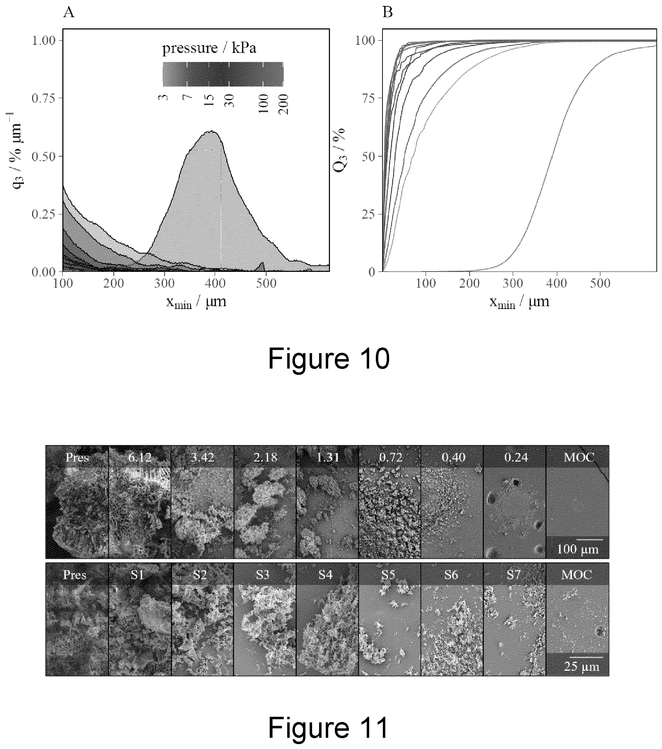

The breaking behavior of spray-freeze-dried (SFD) and spray-dried (SD) particles of a 5 % mannitol SFD formulation and a mannitol SD formulation (Pearlitol® SD200) was compared by measuring the particle density distribution and cumulative density distribution in free fall and with dispersion pressures between 3 and 200 kPa applied (see Figure 10). The median particle size in free fall is 399 $\mu$m (grey area (Figure 10A), and cumulative presentation as a dark line (Figure 10B)) with a high average sphericity of 0.87. Applying 3 kPa dispersion pressure led to a breakage of the particles, with median particle size of 111 $\mu$m and a sphericity of 0.68. A further increase in dispersion pressures led to distinct further decrease of the median particle sizes (Figure 10). The figure shows the particle size distributions of the spray-freeze dried particles in free fall and their breaking behavior under dispersion pressure. On the left (A) the particle density plot, while on the right side (B) the cumulative particle size distribution of the same measurement is shown, which provides the percentage of all particles smaller than a specific size. While the general reduction of the particle size distribution in Figure 10A is shown by a significant shift of the distribution to the left, the cumulative presentation of the data also allows an easy estimation of the shift of median particle sizes to smaller values (Figure 10B).

Pearlitol® 200, a spray-dried mannitol on the other hand, exhibited no decrease in particle size when 3 kPa pressure was applied (median particle size 134 $\mu$m and sphericity 0.825 in free fall and 138 $\mu$m and 0.809 respectively, not shown in Figure 10). This underlines the ability of spray-freeze-dried powder formulations to pulverize in a gas flow. In Figure 10, the particle size reduction with increasing dispersion pressure is clearly visible.

Example 9: SEM imaging of NGI stages

The fragmentation process of samples of various NGI stages of powders according to the present disclosure were electron microscopically imaged (Figure 11, Mannitol with Fluorescein at 5 % solid content). Very small fragments (<5 $\mu$m) were observed at all stages, while larger fragments were increasingly deposited on earlier stages. Only a few intact particles were found in the pre-separator. Broken but partially intact particle structures with geometric diameters of up to 150 $\mu$m were observed up to the second stage, indicating that the corresponding aerodynamic diameter is only 3 % of the geometric one. Smaller fragments of intact particles were occasionally observed on stage 3 and up to stage 4. Only increasingly smaller agglomerates of fragments were detected in stages 5 and above. Very little sample material was found in the MOC, with the fragments found there being <1 $\mu$m in size. Only a few particles undergo partial breakage. Mostly, they tend to fully pulverize and then agglomerate explaining the observed good acceleration capability. The fragments of the particles result in diameters between 3 and 7 $\mu$m, corresponding to the length of the solid lamellar structures within the particles. The fragmentation tends to occur up to the size of maximum mechanical stability, exceeding the size range typically found in the MOC.

Pulmonary deposition was found to be dependent from the mechanically breakable properties of the SFD particle components as easily identifiable from factor $f_b$ (Equation 1). This was found for excipients used in the SFD formulations, comprising mannitol, lactose, gelatine, and maltodextrin.

Furthermore, pulmonary deposition has shown to increase with decreasing geometric density and solid content in combination with increasing share of such mechanically breakable components, like mannitol.

Furthermore, pulmonary deposition of an active pharmaceutical ingredient can be increased with SFD particles by generally reducing the solid content of the solution, thus lowering the particle density.

The interplay of these parameters can be used to optimize the pulmonary deposition by balancing the amount of mechanically breakable excipients used for the formulation with the mechanically breakable properties of the active and the total solid content in the spray solution.

EMBODIMENTS

The present disclosure also relates to the following numbered embodiments.

1. A spray-freeze-dried pharmaceutical powder for inhalation, the powder comprising solid particles of at least one excipient and at least one active agent;

wherein the maximum of the particle size distribution of the particles is more than 150 $\mu$m; and

wherein the particles are fragmented during inhalation to pulmonary particles with a median aerodynamic diameter of below 10 $\mu$m.

2. The powder according to embodiment 1, wherein the maximum of the particle size distribution is more than 200 $\mu$m, preferably more than 300 $\mu$m, more preferably more than 350 $\mu$m and/or less than 1000 $\mu$m, preferably less than 800 $\mu$m, more preferably less than 600 $\mu$m and most preferably less than 450 $\mu$m.

3. The powder according to any one of the previous embodiments, wherein the particles are fragmented during inhalation to pulmonary particles with a median aerodynamic diameter of below 8 $\mu$m, preferably below 5 $\mu$m.

4. The powder according to any one of the previous embodiments, wherein the particles have an angle of response below 45 °.

5. The powder according to any one of the previous embodiments, wherein the at least one excipient is selected from the group consisting of gelatine, glucidex, trehalose, lactose, mannitol and mixtures thereof, preferably wherein the at least one excipient is mannitol.

6. The powder according to any of the preceding embodiments, wherein the at least one excipient is present in an amount of at least 80 % w/w, preferable at least 85 % w/w, most preferably at least 88 % w/w, based on the total weight of the powder.

7. The powder according to any one of the preceding claims, wherein the at least one active agent is present in an amount of 20 % w/w or less, preferably 15 % w/w or less, more preferably 12 % w/w or less, based on the total weight of the powder.

8. The powder according to any one of the previous embodiments, wherein the active agent is selected from the group consisting of bronchodilators, corticosteroids, antibiotics, mucolytics, antivirals, antifungals, anti-inflammatory agents and vaccines or biologics designed for pulmonary delivery.

9. The powder according to any one of the previous embodiments, wherein the active agent is selected from Salbutamol, Salmeterol, Formoterol, Terbutaline, Ipratropium bromide, Tiotropium bromide, Budesonide, Fluticasone, Beclomethasone, Mometasone, Ciclesonide, Tobramycin, Aztreonam, Ciprofloxacin, Colistin, Levofloxacin, Acetylcysteine, Dornase alfa, Zanamivir, Ribavirin, Amphotericin B, Voriconazole, Cromolyn sodium, Montelukast, Nedocromil sodium, Palivizumab, Dupilumab, Adalimumab, Infliximab, Influenza vaccine, COVID-19 vaccines, inhalable insulin (Afrezza), Fentanyl, Nitric oxide, or Treprostinil.

10. The powder according to embodiment 10, wherein the active agent is Salbutamol.

11. The powder according to any one of the previous embodiments, wherein the active agent is insoluble in water.

12. The powder according to embodiment 11, wherein the active agent is Aprepitant.

13. The powder according to any one of the previous embodiments, wherein the powder has a bulk density in a range of between 0.01 g/ml to 0.25 g/ml.

14. The powder according to embodiment 13, wherein the bulk density is below 0.15 g/ml, preferably below 0.10 g/ml, more preferably below 0.070 g/ml, even more preferably below 0.050 g/ml, and most preferably below 0.030 g/ml.

15. The powder according to any one of the previous embodiments, wherein the calculated geometric density of the solid particles is in a range between 0.03 and 0.25 g/ml, preferably between 0.04 and 0.15 g/ml, more preferably between 0.05 and 0.1 g/ml, most preferably between 0.04 and 0.08 g/ml.

16. The powder according to embodiment 15, wherein the calculated geometric density is 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24 or 0.25 g/ml.

17. The powder according to embodiment 15, wherein the calculated geometric density is in the range of between 0.03 and 0.07 g/ml, preferably wherein the calculated geometric density is 0.05 g/ml.

18. The powder according to the present invention, wherein the powder is obtained by spray-freeze drying from a sprayable solution with a viscosity in the range of from 1 to about 100 mPa×s.

19. The powder according to embodiment 18, wherein the sprayable solution is aqueous.

20. The powder according to embodiment 18, wherein the sprayable solution is based on an organic solvent, preferably selected from the group consisting of TBA and/or DMSO.

21. The powder according to any one of the previous embodiments, wherein the fine particle fraction (FPF) of the pulmonary particles after fragmentation is more than 30 %, preferably more than 40 %, even more preferably more than 50 %, most preferably more than 60 %.

22. The powder according to any one of the previous embodiments, wherein the particles undergo fragmentation by inhalation to pulmonary particles without the need of a further modified inhalation device that triggers fragmentation.

23. The powder according to any one of the previous embodiments, wherein the fragility factor $f_b$ is $\geq 0.25$ and the cohesion factor $f_c \leq 0.17$.

24. The powder according to any one of the previous embodiments for use in the treatment of pulmonary diseases.

25. The powder according to embodiments 24 for use in the treatment of Asthma, Chronic Obstructive Pulmonary Disease (COPD), Pulmonary Fibrosis, Cystic Fibrosis, Pulmonary Hypertension, Bronchiectasis, Pneumonia, Tuberculosis (TB), Acute Respiratory Distress Syndrome (ARDS), Lung Cancer, Pulmonary Embolism (PE), Sarcoidosis, Interstitial Lung Disease (ILD), Alpha-1 Antitrypsin Deficiency, Bronchiolitis Obliterans (Popcorn Lung), Hypersensitivity Pneumonitis, Pleural Effusion, Asbestosis, Respiratory Syncytial Virus (RSV) Infection.

26. The powder for use according to embodiment 24 or 25, wherein the spray-freeze-dried pharmaceutical pulmonary powder is dispersed by a passive of a dry powder inhaler.

27. The powder for use according to any one of embodiments 24 andc 25, wherein the spray-freeze-dried pharmaceutical pulmonary powder is dispersed by an active type of a dry powder inhaler.

28. A capsule suitable for provision in an inhalation system comprising the powder according to any of the embodiments 1 to 23.

29. A capsule according to embodiment 28, wherein the capsule is made from a biocompatible material, preferably from gelatin, hydroxypropyl methylcellulose (HPMC), pullulan, or starch-based materials.

**Claims**

1.  A spray-freeze-dried pharmaceutical powder for inhalation, the powder comprising solid particles comprising at least one excipient and at least one active agent;

    wherein the maximum of the particle size distribution of the particles is more than 150 $\mu$m; and
    wherein the particles are fragmented during inhalation to pulmonary particles with a median aerodynamic diameter of below 10 $\mu$m.

2.  The powder according to claim 1, wherein the maximum of the particle size distribution of the particles is more than 200 $\mu$m, preferably more than 300 $\mu$m, more preferably more than 350 $\mu$m and/or less than 1000 $\mu$m, preferably less than 800 $\mu$m, more preferably less than 600 $\mu$m and most preferably less than 450 $\mu$m.

3.  The powder according to any one of the preceding claims, wherein the at least one excipient is selected from the group consisting of gelatine, maltodextrin, trehalose, lactose, mannitol or mixtures thereof, preferably mannitol.

4.  The powder according to any of the preceding claims, wherein the at least one excipient is present in an amount of at least 80 % w/w, preferable at least 85 % w/w, most preferably at least 88 % w/w, based on the total weight of the powder.

5.  The powder according to any one of the preceding claims, wherein the at least one active agent is present in an amount of 20 % w/w or less, preferably 15 % w/w or less, more preferably 12 % w/w or less, based on the total weight of the powder.

6.  The powder according to any one of the preceding claims, wherein the active agent is selected from the group consisting of bronchodilators, corticosteroids, antibiotics, mucolytics, antivirals, antifungals, anti-inflammatory agents and vaccines or biologics designed for pulmonary delivery.

7.  The powder according to any one of the preceding claims, wherein the active agent is selected from Salmeterol, Formoterol, Terbutaline, Ipratropium bromide, Tiotropium bromide, preferably the active agent is salbutamol.

8.  The powder according to any one of the preceding claims, wherein the powder has a bulk density in a range between 0.01 g/ml to 0.25 g/ml.

9.  The powder according to any one of the preceding claims, wherein the calculated geometric density of the solid particles is in a range between 0.03 and 0.25 g/ml, preferably between 0.04 and 0.15 g/ml, more preferably between 0.05 and 0.1 g/ml, most preferably between 0.04 and 0.08 g/ml.

10. The powder according to any one of the preceding claims, wherein the fine particle fraction (FPF) of the pulmonary particles after fragmentation is more than 30 %, preferably more than 40 %, even more preferably more than 50 %, most preferably more than 60 %.

11. The powder according to any one of the preceding claims, wherein the particles undergo fragmentation by inhalation to pulmonary particles without the need of a further modified inhalation device that triggers fragmentation.

12. The powder according to any one of the preceding claims, wherein the fragility factor $f_b$ is $\geq 0.25$ and the cohesion factor $f_c$ is $\leq 0.17$.

13. The powder according to any one of the preceding claims for use in the treatment of a pulmonary disease.

14. The powder for use according to claim 13, wherein the spray-freeze-dried pharmaceutical pulmonary powder is dispersed by a passive type of a dry powder inhaler, or by an active type of a dry powder inhaler.

15. A capsule suitable for provision in an inhalation system comprising the powder according to any of the claims 1 to 12.

Figure 1

Figure 2

Figure 3

Figure 4

Figure 5

Figure 6

Figure 7

# Figure 8A and B

# Figure 9

Figure 10

Figure 11

Figure 12

Figure 13

Figure 14

Figure 15

Europäisches
Patentamt

European
Patent Office

Office européen
des brevets

# EUROPEAN SEARCH REPORT

**Application Number**

EP 24 21 6569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANNING STEFAN ET AL: "Impact of excipient choice on the aerodynamic performance of inhalable spray-freeze-dried powders", INTERNATIONAL JOURNAL OF PHARMACEUTICS, ELSEVIER, AMSTERDAM, NL, vol. 586, 23 June 2020 (2020-06-23), XP086242277, ISSN: 0378-5173, DOI: 10.1016/J.IJPHARM.2020.119564 [retrieved on 2020-06-23] | 1-6,8-15 | INV. A61K9/72 A61K47/10 A61K47/26 A61K47/32 A61K47/42 A61K31/137 A61K31/167 A61K31/439 A61K31/46 A61K31/5377 A61K9/16 |
| Y | * the whole document * <br> * abstract * <br> * figures * <br> * Materials and Methods * <br> ----- | 7 | |
| X | WANNING STEFAN ET AL: "Aerodynamic Droplet Stream Expansion for the Production of Spray Freeze-Dried Powders", AAPS PHARMSCITECH, SPRINGER US, NEW YORK, vol. 18, no. 5, 19 October 2016 (2016-10-19), pages 1760-1769, XP036266280, DOI: 10.1208/S12249-016-0648-2 [retrieved on 2016-10-19] | 1-6,8-15 | |
| Y | * the whole document * <br> * abstract * <br> * tables * <br> * figures * <br> ----- <br> -/-- | 7 | |

**TECHNICAL FIELDS SEARCHED (IPC)**

A61K

The present search report has been drawn up for all claims

| Place of search | Date of completion of the search | Examiner |
|---|---|---|
| The Hague | 25 April 2025 | Marchand, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

**EP 4 751 719 A1**

# EUROPEAN SEARCH REPORT

**Application Number**

EP 24 21 6569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEN YINGJIE ET AL: "Micro-fluidic Spray Freeze Dried Ciprofloxacin Hydrochloride-Embedded Dry Powder for Inhalation", AAPS PHARMSCITECH, SPRINGER INTERNATIONAL PUBLISHING, CHAM, vol. 23, no. 6, 2 August 2022 (2022-08-02), XP037925272, DOI: 10.1208/S12249-022-02371-0 [retrieved on 2022-08-02] | 1,2,4-6, 8-15 | |
| Y | * the whole document *<br>* abstract *<br>* figures *<br>* Materials and Methods * | 7 | |
| X | WO 2013/041542 A1 (LAMPRECHT ALF [DE]) 28 March 2013 (2013-03-28) | 1-6,8-15 | |
| Y | * the whole document *<br>* page 1, line 16 - line 18 *<br>* page 2, line 18 - page 4, line 23 *<br>* examples *<br>* tables *<br>* claims * | 7 | |
| X | RAUTENBERG ANNIKA ET AL: "Spray-freeze-dried lyospheres: Solid content and the impact on flowability and mechanical stability", POWDER TECHNOLOGY, ELSEVIER, BASEL (CH), vol. 411, 6 September 2022 (2022-09-06), XP087192520, ISSN: 0032-5910, DOI: 10.1016/J.POWTEC.2022.117905 [retrieved on 2022-09-06] | 1-6,8-15 | TECHNICAL FIELDS SEARCHED (IPC) |
| Y | * the whole document *<br>* abstract *<br>* Experimental * | 7 | |

-/--

The present search report has been drawn up for all claims

| Place of search | Date of completion of the search | Examiner |
|---|---|---|
| The Hague | 25 April 2025 | Marchand, Petra |

EPO FORM 1503 03.82 (P04C01)

page 2 of 3

Europäisches
Patentamt

European
Patent Office

Office européen
des brevets

**EUROPEAN SEARCH REPORT**

Application Number

EP 24 21 6569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CA 2 585 314 A1 (UNIV GRONINGEN [NL]; SOLVAY PHARM BV [NL]) 18 May 2006 (2006-05-18) | 1,2,4-6, 8-15 | |
| Y | * the whole document * <br> * examples * <br> * table 2 * <br> * figures * <br> * claims * <br> ----- | 7 | |
| Y | WANNING STEFAN ET AL: "Pharmaceutical spray freeze drying", INTERNATIONAL JOURNAL OF PHARMACEUTICS, ELSEVIER, AMSTERDAM, NL, vol. 488, no. 1, 18 April 2015 (2015-04-18), pages 136-153, XP029235867, ISSN: 0378-5173, DOI: 10.1016/J.IJPHARM.2015.04.053 * the whole document * * section 3.2.1 * ----- | 7 | |
| | | | **TECHNICAL FIELDS SEARCHED (IPC)** |

The present search report has been drawn up for all claims

| Place of search | Date of completion of the search | Examiner |
|---|---|---|
| The Hague | 25 April 2025 | Marchand, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

EP 4 751 719 A1

## ANNEX TO THE EUROPEAN SEARCH REPORT
## ON EUROPEAN PATENT APPLICATION NO.

EP 24 21 6569

This annex lists the patent family members relating to the patent documents cited in the above-mentioned European search report.
The members are as contained in the European Patent Office EDP file on
The European Patent Office is in no way liable for these particulars which are merely given for the purpose of information.

25-04-2025

| Patent document cited in search report | | Publication date | Patent family member(s) | | Publication date |
|---|---|---|---|---|---|
| WO 2013041542 | A1 | 28-03-2013 | NONE | | |
| CA 2585314 | A1 | 18-05-2006 | AR | 052787 A1 | 04-04-2007 |
| | | | CA | 2585314 A1 | 18-05-2006 |
| | | | CN | 101056622 A | 17-10-2007 |
| | | | EP | 1811965 A1 | 01-08-2007 |
| | | | JP | 5137579 B2 | 06-02-2013 |
| | | | JP | 2008519806 A | 12-06-2008 |
| | | | TW | 200621310 A | 01-07-2006 |
| | | | WO | 2006051067 A1 | 18-05-2006 |

EPO FORM P0459

For more details about this annex : see Official Journal of the European Patent Office, No. 12/82

32

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **S. M. MAYRAMNEGARI et al.** *Journal of Food Engineering*, 2023, vol. 338